# EUROPEAN PATENT SPECIFICATION

(11) **EP 0 759 944 B1**
(45) Date of publication and mention of the grant of the patent: **16.08.2001**
(21) Application number: 95918692.5
(22) Date of filing: 15.05.1995
(51) Int. Cl.: C07K 19/00, A61K 39/00

(54) **Target cell binding chimaeric peptides**
Zielzellen-bindende chimäre Peptide
Peptides chimèriques liants des cellules cibles

(30) Priority: 13.05.1994 GB 9409643; 31.08.1994 GB 9417461
(43) Date of publication of application: 05.03.1997
(73) Proprietor: Biovation Limited, Aberdeen AB22 8GU (GB)
(72) Inventor: CARDY, Donald Leonard Nicholas, Aston-le-Walls Northamptonshire NN11 6UF (GB); CARR, Frank Joseph, Balmedie Aberdeenshire AB23 8XU (GB)
(74) Representative: Bassett, Richard Simon
(86) International application number: GB9501107
(87) International publication number: WO9531483

(56) References cited:
- EP-A- 0 532 090
- EP-A- 0 541 335
- WO-A-95/11977
- PROCEEDINGS OF THE NATIONAL ACADEMY OF SCIENCES OF THE USA, vol. 90, no. 8, 15 April 1993 WASHINGTON, DC, USA, pages 3530-3534, J. DONNELLY ET AL. 'Targeted delivery of peptide epitopes to class I major histocompatibility molecules by a modified Pseudomonas exotoxin.' cited in the application
- THE JOURNAL OF IMMUNOLOGY, vol. 152, no. 5, 1 March 1994 BALTIMORE, MD, USA, pages 2377-2384, C. SIEGALL ET AL. 'In vitro and in vivo characterization of BR96 sFv-PE40.'
- SCIENCE, vol. 261, no. 5118, 9 July 1993 WASHINGTON, DC, USA, pages 212-215, P. TRAIL ET AL. 'Cure of xenografted human carcinomas by BR96-doxorubicin immunocomjugates.'
- IMMUNOLOGY, vol. 81, no. 1, January 1994 OXFORD, GB, pages 167-170, A. SCARDINO ET AL. 'Antigen targeting to antigen-presenting cells enhances presentation to class II-restricted T lymphocytes.'
- CLINICAL AND EXPERIMENTAL IMMUNOLOGY, vol. 97, no. 3, September 1994 OXFORD, GB, pages 361-366, S. PEIFANG ET AL. 'Enhanced activation of human T cell clones specific for virus-like particles expressing the HIV V3 loop in the presence of HIV V3 loop-specific polyclonal antibodies.'

### SEQUENCE LISTING

(1) GENERAL INFORMATION:
   (i) APPLICANT:
      (A) NAME: ECLAGEN LIMITED
      (B) STREET: Marischal College. Broad Street
      (C) CITY: Aberdeen
      (E) COUNTRY: United Kingdom
      (F) POSTAL CODE (ZIP): AB9 1AS
      (G) TELEPHONE: (01224) 273002
      (H) TELEFAX: (01224) 273198
   (ii) TITLE OF INVENTION: Improvements in or Relating to Peptide Delivery
   (iii) NUMBER OF SEQUENCES: 6
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 2:
   CATGCAGGAT GAGTTGCAGA TATACTTCGC GGCTGC 36
(2) INFORMATION FOR SEQ ID NO: 3:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 3:
   GCAGCCGCGG GAATACTAGG GTTCGTATTT ACACTA 36
(2) INFORMATION FOR SEQ ID NO: 4:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 4:
   TAGTGTAAAT ACGAACCCTA GTATTCCCGC GGCTGC 36
(2) INFORMATION FOR SEQ ID NO: 5:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 5:
   GGCCGCTACG GACGAAAGAA GAGGCGTCAA CGACGCAGAC CA 42
(2) INFORMATION FOR SEQ ID NO: 6:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 6:
   TGGTCTGCGT CGTTGACGCC TCTTCTTTCG TCCGTAGCGG CC 42

## Description

### Field of the Invention

This invention relates to chimaeric polypeptides designed to bind to a target cell, to vaccines comprising the chimaeric polypeptides, and to a method of modulating the immune response of a human or animal subject.

In particular, the invention relates to chimaeric polypeptides designed for the efficient delivery of one or more effector molecules to a target cell for subsequent internalisation by the cell. In specific embodiments the invention relates to recombinant antibody molecules containing immunodominant peptide sequences whereby these antibody molecules enter cells via internalising antigens or receptors and are subsequently processed to release peptides for antigen presentation leading to either induction or inhibition of an immune response. The invention also relates to methods for treatment and prevention of disease using these new types of recombinant antibodies or proteins and methods for generating these molecules.

### Background of the Invention

The finding that T-cells recognise antigen as peptides bound to major histocompatability complex (MHC) molecules has given rise to attempts to use defined synthetic peptides corresponding to immunodominant T-cell epitopes to either activate or tolerise T-cells to specific antigens. Generally, the approach used has been to immunise with the immunogenic peptide in order to induce a T-cell response relying on the efficient processing and antigen presentation by professional immune system cells to induce a T-cell response comprising either a predominant response by CD8+ cytotoxic T lymphocytes (CTLs) for peptide presented in conjunction with MHC class I molecules or a predominant CD4+ helper T-cell response for peptide presented in conjunction with MHC class II molecules. For the treatment of cancer, there is now a major interest in the induction (or adoptive transfer) of CTLs specific for tumour-associated antigens such as the MAGE-1 antigen produced by a high proportion of human melanoma tumours (van der Bruggen *et al.,* 1991 Science *254*, 1643). There is also an interest in the identification of peptides associated with diseases such as autoimmunity in order to induce tolerance by administration of the autoimmune peptide and subsequent presentation and overstimulation leading to abrogation of peptide-specific CTLs (e.g. Aichele *et al*., 1994 PNAS *91*, 444). There is additionally an interest in the identification of peptides associated with diseases such as allergy in order to redirect a TH2 response induced by the allergen to a TH1 response by immunising with a peptide homologue of the TH1-inducing epitope. Another approach is to immunise with altered peptides which, upon presentation, can bind to but not activate a specific T-cell thus antagonising deleterious responses of such T-cells.

The present inventors consider that there are three main limitations to the direct use of peptides for activation or tolerance of specific T-cells to antigens involved in disease processes. Firstly, peptide-induced activation of cytotoxic T cells for the subsequent destruction of aberrant disease-associated target cells is restricted to target cells which present the specific peptide, usually professional (constitutive) or facultative antigen presenting cells and also cells expressing abnormal antigens such as cancer cells expressing the MAGE-1 antigen. Thus, it is currently not possible to direct the activation of T cells by antigen presentation by specifically chosen cell types. Secondly, peptide-induced activation or tolerance relies on the efficient uptake and presentation of peptide by professional or facultative antigen presenting cells; the efficiency of presentation is highly dependent on factors such as peptide concentration and peptide formulation with adjuvants which are variable for different peptides and do not always result in a specific T-cell response. Thirdly, peptide presentation is MHC-restricted and dependent on specific HLA type and thus the activation or tolerance of T cells to a certain immunodominant peptide is dependent on the HLA type of the individual. Moreover, exogenously added peptides (such as used in vaccines) will normally be taken up and processed by cells in such a way that they are presented to the immune system in association with MHC class II antigens only - exogenously added peptides tend not to enter the processing pathway which leads to association with MHC class I antigens, which association is essential for eliciting a CTL response. This problem has not so far been satisfactorily addressed.

Siegall *et al* (1994) *J Immunol* **152**,2377-2384 describes a fusion protein containing three components: a translocation/effector portion from *Pseudomonas* endotoxin A and a binding portion derived from an immunoglobulin molecule.

Scardino *et al* (1994) *Immunol* **81**,167-170 describes a hybrid molecule containing the HIV-derived gp 120 molecule as a binding portion coupled to an antigen (HBenvAg). No translocation portion is mentioned.

### Summary of the Invention

The invention provides a chimaeric polypeptide comprising a binding portion having specific binding affinity for a eukaryotic target cell surface component, and an effector portion consisting of one or more copies of an immunogenic peptide; whereby binding of the polypeptide to the cell surface component induces internalisation of at least the effector portion so as to allow the immunogenic peptide to be presented by MHC molecules on the target cell surface.

The chimaeric polypeptide may comprise a binding portion, from a first source having specific binding affinity for a eukaryotic target cell surface component and an effector portion from a second source comprising an amino acid sequence (peptide) capable of exerting a biological effect; and a translocation portion, from a third source, the translocation portion being adjacent to the effector portion; whereby binding of the polypeptide to the cell surface component induces internalisation of at least the effector and translocation portions so as to allow the effector portion to enter the cytosol of the target cell and thence allow the peptide to exert its biological effect.

Typically, the whole of the chimaeric polypeptide will be internalised, but this is not essential.

The binding portion comprises an immunoglobulin molecule (e.g. an antibody) or an effective portion thereof.' The term "effective portion" is intended to denote those portions of immunoglobulin molecules which retain some degree of specific binding affinity, and includes Fab, Fv, SCA and scFv fragments.

The biological effect mediated by the amino acid sequence will be an immunomodulatory effect. Preferably the amino acid sequence capable of exerting an immunomodulatory effect will be an immunodominant peptide, typically a T cell epitope. The effector portion may comprise a single amino acid sequence capable of exerting an immunomodulatory effect or may comprise a plurality of such sequences. Where the effector portion of the chimaeric polypeptide comprises a plurality of such amino acid sequences, they may be joined to form a single "domain"-like structure or may be disposed at different points in the polypeptide. Similarly, where the effector portion comprises a plurality of amino acid sequences, they may be repeats of the same sequence or may be single copies of various different amino acid sequences.

Conveniently the effector portion will also comprise a signal which directs the amino acid sequence to a specific cellular compartment. Such signals are further discussed below.

The invention also provides a vaccine comprising the chimaeric polypeptide defined above, and a method of regulating the immune response of a human or animal subject. The invention also includes within its scope a method of making the chimaeric polypeptide defined above.

Accordingly, instead of relying on individual peptides being taken up and presented by professional or facultative antigen-presenting cells, the present invention uses antibody or protein molecules specific for internalising antigens or receptors on any cell type to deliver one or more peptides internally to these cells. Upon digestion and processing of the recombinant antibody/protein-peptide molecules, the peptides can then be transported to and presented on the cell surface by appropriate MHC molecules or can interact with other intracellular molecules and remain within the cell. In particular, the present invention relates to a new class of antibody or protein molecule produced by recombinant DNA methods to include within the protein the sequence of one or more immunodominant peptides. Thus the invention provides, for the first time, a means of targeting antigen presentation to a particular chosen cell with the main requirement of an internalising antigen or receptor bound by an antibody or protein containing the peptide antigen to be presented (optimally in multiple copies).

Advantages of the present invention for the activation or tolerance of T-cells are several-fold. Firstly, by using an antibody or protein to deliver immunodominant peptides, these peptides can be delivered, via an internalising antigen, to cells which do not normally present such peptides. In the case of cancer cells for example, a cancer cell can then present one or more peptides designed to activate CTLs to subsequently destroy the cancer cell. In the case of normal cells subject to autoimmune destruction by CTLs, these peptides can be delivered via an internalising antigen or receptor either as high doses of normal immunodominant peptides to induce tolerance by abrogation of the usually destructive CTLs or as altered peptides which antagonise the disease-associated T-cell activation. Secondly, several different peptide sequences or multiple copies of the same peptide sequence can be associated with a single antibody or protein molecule, thus providing either the possibility for a choice of peptides for presentation by the appropriate MHC haplotype in the individual immunised or the possibility for efficiently delivering a high dose of peptide via multiple copies on the antibody molecule. Thirdly, by using an antibody or protein to deliver immunodominant peptides, these peptides can be efficiently delivered, via an internalising antigen, to specific professional antigen presenting cells as an alternative to the less specific endocytosis by these cells; specific antigen-presenting cells can thus he targeted in order to better control the type of immune response to the immunodominant peptide.

It will be understood that antibodies or proteins of the present invention can be used to deliver peptides into cells, either for subsequent binding to and presentation by MHC molecules, or for binding to specific intracellular molecules to produce effects such as blocking intracellular protein-protein interactions (such as those involved in intracellular signalling). It will be understood that the present invention can apply to both class-I and class-II MHC-restricted immunodominant peptides and can potentially be applied to the targeted induction of an immune response to many different antigens in many different disease states such as cancer (induction of CTLs by cancer cells), autoimmunity (antagonism of T-cell activation or induction of tolerance), infection (induction of CTLs by infected cells, antagonism of T helper cell activation by superantigen), allergy (diversion of a TH2 to a TH1 response by the immunodominant peptide) and inflammation (antagonism of T-cell activation). It will also be understood that antibodies or proteins produced by the present invention could include the so-called "universal" immunodominant peptides (e.g. tetanus toxin and influenza nucleoprotein peptides) against which a high proportion of individuals might already be sensitised to by vaccination or infection. It will also be understood that, for efficient presentation of MHC class I-associated peptides by antibodies or proteins produced by the present invention, the inclusion in the molecule of sequences facilitiating entry of these peptides into the cytoplasm from compartments such as the endosomes or trans golgi network may be required and that certain such sequences (or domains) already known in the literature (such as the translocation domains of toxins such as Pseudomonas exotoxin (Donnelly et al., 1993 PNAS *90*, 3530), the HIV-1 tat protein (Fawell *et al*., 1994 PNAS *91*, 664) or the endosome-disrupting functions of viruses such as adenovirus (Curiel *et al*., 1991 PNAS *88*, 8850). It will also be understood that antibodies or proteins produced by the present invention could include a mixture of different peptides which associate with different MHC class I or class II molecules thus providing for a broader prospect of inducing an immune response in different population groups carrying different HLA types. It will also be understood that the antibodies or proteins of the present invention could be used in several different ways in disease treatment and prevention including to deliver peptides *ex vivo* for the induction of CTLs prior to adoptive immunotherapy and to enhance the T-cell activating, blocking or abrogating ability of cells already presenting the specific peptide(s) or analogues thereof. It will be understood that antibodies or proteins of the present invention could be targeted to professional antigen presenting cells such as, for example, macrophages (e.g. via the FcRI receptor) and B cells (e.g. via surface immunoglobulin molecules). Finally, it will be understood that the antibodies or proteins of the present invention might be used for disease treatment or prevention either alone or in combination with other molecules which potentiate the interaction with T-cells such as molecules which up-regulate class I and class II MHC expression in certain cell types such as interferon-gamma or that the antibodies or proteins of the present invention might themselves be used as vaccines through the inclusion of adjacent immunodominant B and T-cell epitopes optimally as multiple copies and targeted to professional antigen presenting cells.

It will be understood by those skilled in the art that a range of internalising antigens might be targeted by the polypeptides of the invention in delivering peptides intracellularly to specific cells. For example, MHC class I or class II molecules might be targeted on professional antigen-presenting cells in order, for example, to promote a strong immune response against the delivered peptides and to deliver the peptides to these cells without use of an adjuvant. In addition, other professional antigen-presenting cells might be targeted such as mucosal cells and dendritic cells and also specific lymphocyte subsets might be targeted through the use of antibodies which bind to lymphocyte cell surface internalising antigens such as surface immunoglobulins (sIg).

It will be understood by those skilled in the art that immunodominant peptides might be included at many different locations in an antibody molecule using recombinant DNA. It will be understood that incorporation or conjugation of these peptides should be such that, as required. antibody function such as binding affinity and rate of clearance is not impaired. Alternatively, it will be understood that peptides could be incorporated as part of an incomplete recombinant antibody fragment such as an Fab, an Fv or an SCA (single chain-antibody) fragment. Depending on the application, it might be preferable to include the immunodominant peptides in a protein domain or structure designed to either mask the peptides from specific recognition by circulating or cell-bound molecules or to expose the peptides such that some immune recognition is possible instead of direct uptake by an internalising antigen. For peptides which particularly stimulate a good B-cell response following internalisation and presentation (particularly MHC class-II restricted), it might be preferable to mask the peptides from specific recognition by circulating antibodies in order to avoid premature clearance by the immune system. Finally, it will be understood that a bivalent (or multivalent) antibody might be used consisting of one part binding to the internalising antigen and another part with no specific binding specificity but including the immunodominant peptide either within or attached to the antibody structure (including immunodominant peptides insertion at the usual sites for CDRs (complementarity-determining regions).

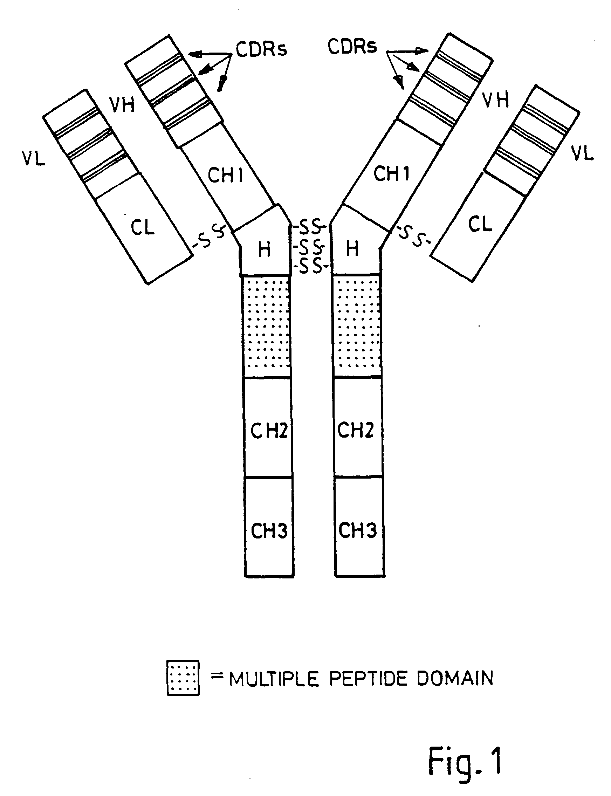

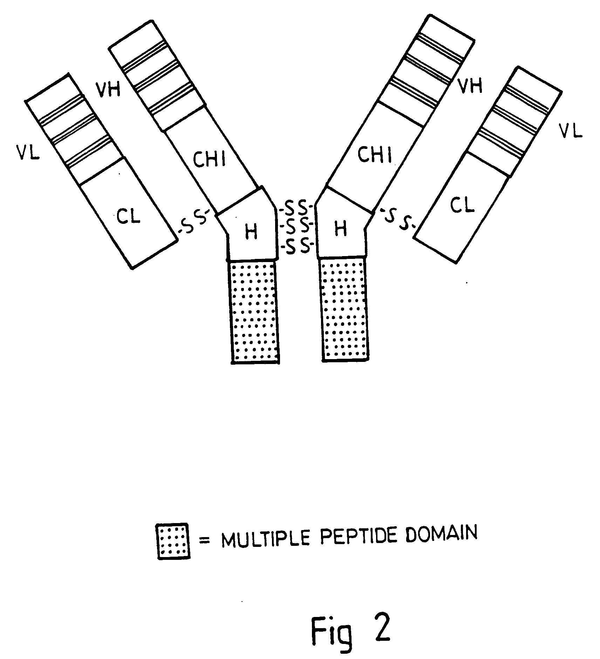

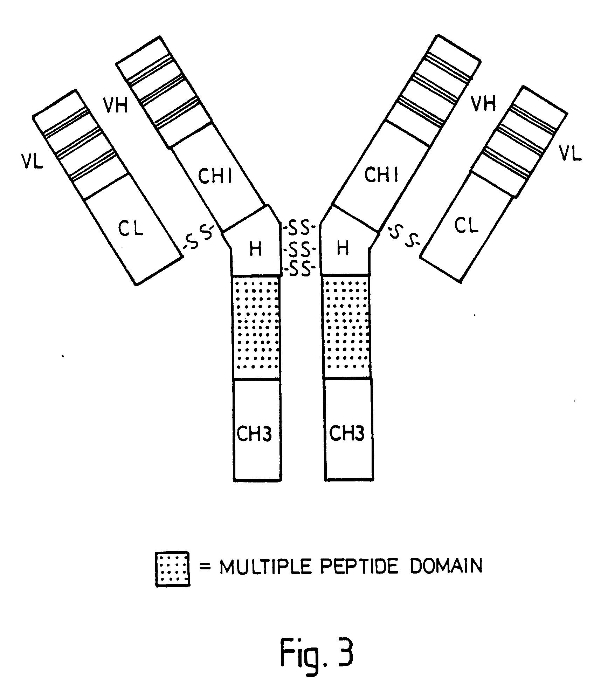

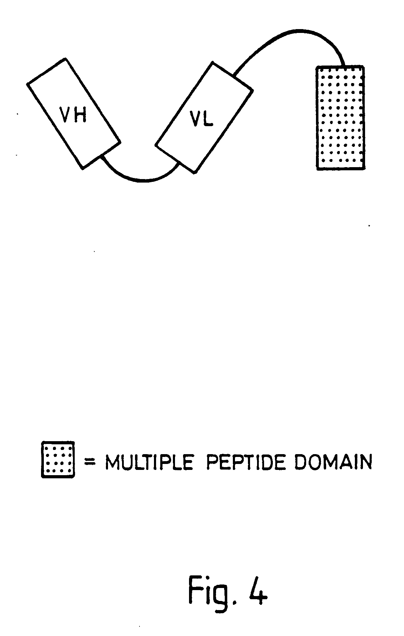

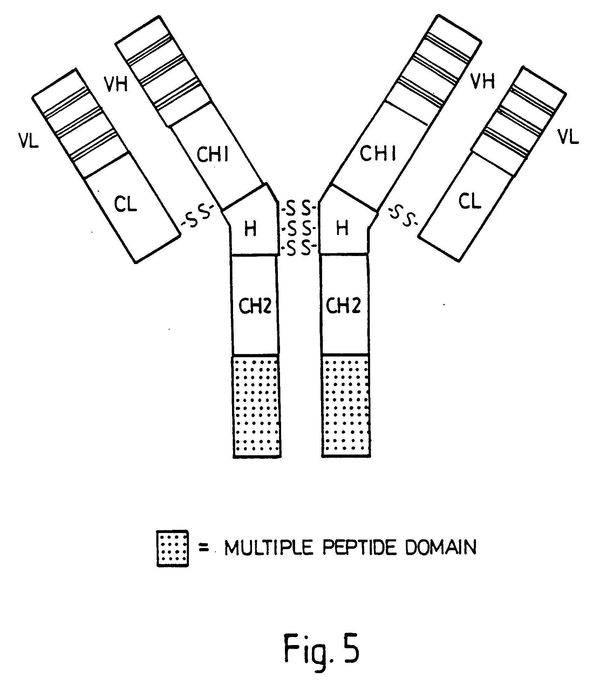

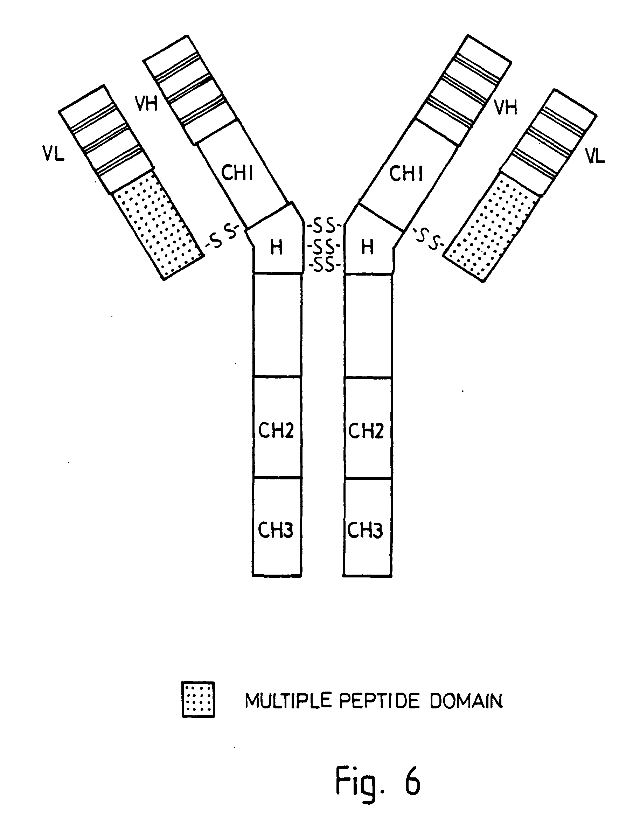

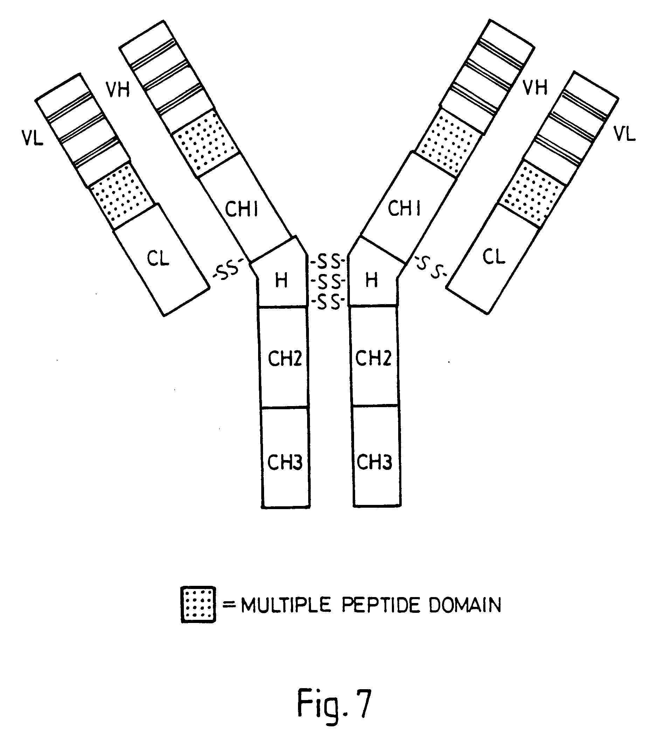

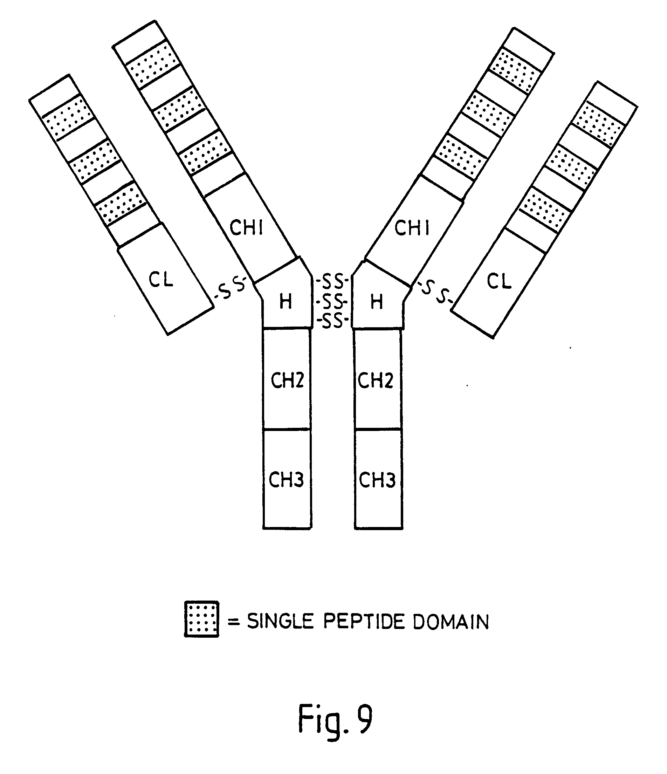

The invention will now be further described by way of illustration and with reference to the accompanying drawings, wherein Figures 1 to 9 illustrate a number of possible designs for antibodies including immunodominant peptides either in discrete multi-peptide domains (Figures 1 to 8) or as individual peptide sequences included to replace CDRs (Figure 9). These Figures should not be considered an exclusive set of designs for antibodies containing immunodominant peptides and should not be considered as limiting the scope of the invention. For these Figures, antibodies are drawn comprising CDRs, VH and VL domains i.e. variable region heavy and light chain domains (including the CDRs), CL domains i.e. light chain constant domains, and CH1-3 (i.e. heavy region constant domains). The portions of the molecule comprising the amino acid sequences capable of exerting a biological effect are shown dotted.

Figure 1 illustrates an antibody whereby the immunodominant peptide domain including multiple copies of the same immunodominant peptide or, alternatively, individual copies of multiple peptides (for example, to compensate for MHC-restriction by providing a range of options covering different MHC haplotypes) has been fused adjacent to the hinge ("H") region in the heavy chains upstream from the CH2 domains;

Figure 2 illustrates an antibody whereby the immunodominant peptide domain has been fused adjacent to the hinge region in the heavy chains thus replacing the CH2 and CH3 domains to create a Fab-peptide hybrid antibody;

Figure 3 this illustrates an antibody whereby the immunodominant peptide domain has been fused adjacent to the hinge region in the heavy chains replacing the CH2 domains;

Figure 4 illustrates a SCA (single-chain antibody) whereby the immunodominant peptide domain has been fused adjacent to a recombinant Fv via a linker arm;

Figure 5 illustrates an antibody whereby the CH3 domain has been replaced by the immunodominant peptide domain;

Figure 6 illustrates an antibody whereby the immunodominant peptide domain has been fused adjacent to the VL region in the light chains thus replacing the CL domains;

Figure 7 illustrates an antibody whereby the immunodominant peptide domain has been fused adjacent between the VH and CH1 domains in the heavy chains and between the VL and CL domains in the light chains;

Figure 8 illustrates a bispecific antibody created by either the coexpression or thiol reduction/oxidation of a mixture of antibody heavy chains whereby in one the immunodominant peptide domain has been fused adjacent to the hinge region thus replacing the CH2 and CH3 domains;

Figure 9 illustrates an antibody whereby 6 copies (or 6 variants) of the immunodominant peptide have been inserted in the VH / VL regions to replace the CDRs; and

Figure 10 illustrates the typical organisation of an immunodominant peptide domain for HLA class I-associating peptides whereby several peptide copies arranged in a contiguous fashion are fused to a translocating domain (D) and optionally a C-terminal KDEL-like sequence.

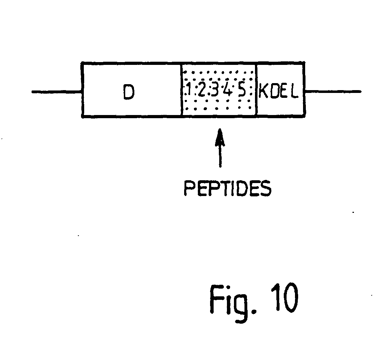

The peptide domains referred to in Figures 1 to 8 and depicted in Figure 10 above could comprise either multiple repeats of the same immunodominant peptide or a range of different immunodominant peptide sequences within the same domain. In each case, the peptide sequences may be repeated in head-to-tail or head-to-head organisation, preferably with suitable amino acid spacing between the sequence repeats provided by non-immunodominant sequences. In each case, the immunodominant peptide content of heavy and light chains might be the same or different and peptide domains may be added to all the chains, or to the two heavy or the two light chains only, or to just one of the heavy and/or light chains or as part of a bispecific (or multispecific) antibody whereby the peptide domains might only be derived from one or more of the recombinant chains used to create the bispecific antibody. It will be apparent that the arrangement of immunodominant peptide sequences in the peptide domain might require optimisation for different peptides included in the domain and might need to be optimised in order to ensure efficient production of the recombinant antibody without disruption of the antibody-like structure, and that such optimisation should be a matter of comparatively simple trial and error for those skilled in the art. It will also be understood that optimisation of antibodies for effective processing of the immunodominant peptide domains might also he required. In each case, optimisation might require different arrangements of peptides in conjunction with each other, different flanking amino acid sequences between the immunodominant peptides (including introduction of sequences, such as acid-labile or peptidase sites, to promote cleavage upon internal processing outside the immunodominant peptide regions) and different combinations with other peptide or protein structures as required for various reasons (e.g. keeping the total molecule from misfolding or to keep the immunodominant peptides either internalised or externalised in a globular protein structure). It will also be apparent that, for HLA class I-associating peptides, the addition of a translocating domain (or domains) adjacent to the tandemly-arranged peptides might be required for efficient transport to, and processing in, the cytoplasm.

The following examples serve to illustrate the possible applications of antibodies of the present invention and should not be considered as limiting the scope of the invention.

### Example 1:

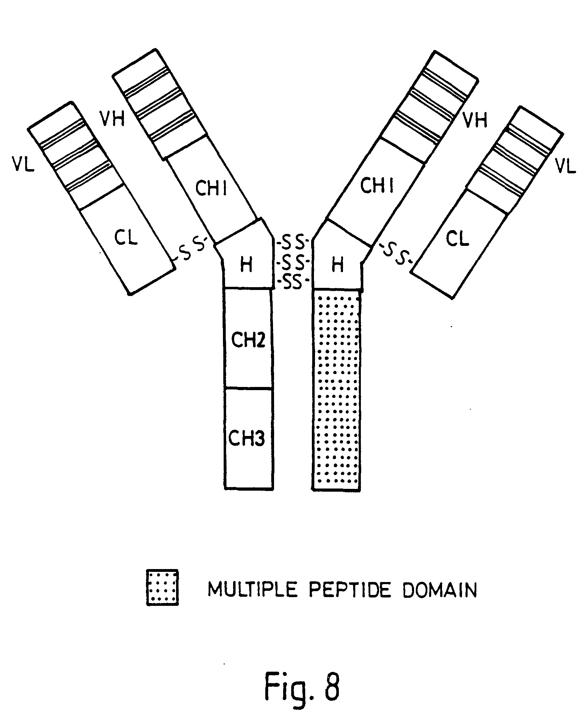

The generation of recombinant monoclonal antibodies suitable for use in treatment and prevention of human disease has been comprehensively described in the literature (for example: Boulianne *et al*., 1984 Nature *312*, 643; Morrison *et al*., 1984 PNAS *81,* 6851; Reichmann *et al*., 1988 Nature *332,* 323; Queen *et al*., 1989 PNAS, p10029) and methods have been developed for production of whole antibodies from mammalian and other eucaryotic cells and also for production of antibody fragments (e.g. Fv, Fab, and SCA fragments) from *E*. *coli*. For generation of antibodies from mammalian cells. all of the antibody designs depicted in Figures 1 to 3 and 5 to 9 can be produced using prior art methods including one or more cloning steps to insert sequences encoding peptides for delivery into cells within the antibody molecule. Methods for generation of bispecific antibodies as depicted in Figure 8 are also known in the art for mammalian cells and bispecific antibody fragments can also be generated from bacteria. By way of example and to illustrate the utility of such antibodies for vaccination, a recombinant antibody was generated which was specific for the invariant chain of the MHC class II antigen on professional antigen-presenting cells. The starting point was the hybridoma cell line DA6.231 obtained from the European Collection of Animal Cell Cultures (ECACC No. 90110606). Cells were grown in RPMI1640 and 10% foetal bovine serum. mRNA isolation and the cloning of scFvs (single-chain Fvs) was by the protocol recommended in a "Recombinant Phage Antibody System" (Pharmacia, Milton Keynes, UK) using reagents in a kit supplied by the manufacturer with cloning into the pCANTAB5E cloning vector and KO7 helper phage (both included in the kit). For isolation of specific scFvs, MHC class II antigen was prepared according to van Heyningen *et al*. (1982 Immunogenetics *16,* 459-469) using the DA6.231 antibody for purification of MHC class II antigen from DAUDI lymphoblastoma cells (ECACC No. 85011437). The soluble antigen preparation derived from 108 cells was absorbed on 3 x T25 tissue culture flasks (Costar) for subsequent panning of phage particles and recovery of residual binding phage particles after three panning steps. Clones of recovered phage were checked for specific binding to MHC class II antigen by subsequent ELISA assays using class II antigen or irrelevant antigens absorbed into 96-well plastic microtitre plates. Peroxidase-labeled anti-M13 antibody was used for analysis as supplied in the Pharmacia kit. An individual phage antibody, termed anti-MHCII, was finally used to infect HB2151 cells to produce soluble recombinant phage antibodies.

All other DNA manipulation steps were performed according to Sambrook et al. (Molecular Cloning, A Laboratory Manual, Cold Spring Harbor Laboratory, Cold Spring Harbor, second edition. 1989). For the insertion of peptide sequences, synthetic oligonucleotides were synthesised using an Applied Biosystems 430A synthesiser and purified by reverse phase HPLC. Sequences were as follows (Seq ID No.s 1 to 6 respectively);

The oligonucleotides P53-U and -L (encoding the p53 CTL reactive peptide KYICNSSCM (Noguchi *et al*., 1994 Proc. Natl. Acad. Sci. USA *91,* 3171-3175) and FLU-U and -L (encoding the influenza A matrix protein peptide GILGFVFTL reactive with influenza-specific CTLs (Gammon *et al*., 1992 J Immunol. *148,* 7-12) were 5'-labelled with ³²P using polynucleotide kinase and ³²P ATP and annealed together, self-ligated at 37°C for 4 hours using T4 DNA ligase (Life Technologies, Paisley UK) and analysed on a preparative polyacrylamide sequencing gel. The bands at 180 base pairs representing 5 self-ligated copies of P53-U/L or FLU-U/L were purified, ligated to phosphorylated *Not*I linkers (#1127, New England Biolabs, Hitchin, UK) and digested with *Not*I (Pharmacia).

pCANTAB5 anti-MHCII scFv phage DNA was digested with *Not*I (Pharmacia). To some samples, TAT-U and TAT-L oligonucleotides were annealed and ligated directly with the *Not*I-digested vector and used to transform *E*. *coli* TG1 cells. DNA from recombinants carrying the TAT sequences was further digested with *Not*I (anti-MHCII/tat) for subsequent cloning of P53 and FLU pentamers. *Not*I digested anti-MHCII or anti-MHCII/tat was ligated with the purified 180 base pair bands of p53 or flu epitopes and used to transform TG1 cells. Following cloning and checking for binding to MHC class II antigen by ELISA, soluble antibodies were generated by phage infection of HB2151 cells according to instructions with the Recombinant Phage Antibody System.

For preparation of cytotoxic T lymphocytes (CTLs) specific for the p53-derived peptide as above. the *in vivo* mouse peptide immunisation and sensitisation procedure of Noguchi et al. (loc. cit.) was followed to produce long-term CTL lines. For testing of antibodies for ability to induce CTL activity, target mouse Sp2/0 cells (ATCC CRL-1581) were used and maintained in DMEM and 10% foetal bovine serum. For CTL assays, cells at 5 x 10⁵ cells/ml were labeled overnight with 20*µ*Ci (7.4MBq) ⁵¹Cr chromate. Cells were then pelleted. washed in medium and resuspended at 5 x 10⁵ cells/ml in medium plus dilutions of the antibody fragments or 10µg/ml peptide KYICNSSCM ("p53 peptide only") for 4 hours at 37°C. Cells were then repelleted, washed twice in PBS (phosphate-buffered saline) and plated at 5 x 10³ cells in 100µl in RPMI1640 medium plus 10% foetal bovine serum in 24-well plates. 100µl of CTLs were then added to give effector:target ratios of 20:1, 10:1 and 5:1 and incubated for 4 hours at 37°C. After incubation, 100µl of culture supernatant was carefully removed from each well into an eppendorf tube, centrifuged and triplicate 20µl aliquots of supernatant were counted in a scintillation counter. Percent specific release was calculated as [(release by effector cells-spontaneous release)/(maximum release-spontaneous release)] x100. Results were as follows;

| **Treatment** | **% Specific** ^{**51**}**Cr Release at E/T Ratio** | | |
|---|---|---|---|
| | **20:1** | **10:1** | **5:1** |
| Anti MHCII-P53 | 23 | 23 | 17 |
| Anti MHCII-P53/tat | 47 | 43 | 27 |
| Anti MHCII-FLU/tat | 6 | 4 | 2 |
| Anti MHCII (no peptides) | 7 | 5 | 2 |
| p53 peptide only | 46 | 57 | 41 |

These results demonstrated the induction of cell lysis following incubation with antibodies specific for MHC class II molecules and carrying the p53 peptide sequence but not to anti-MHC class II carrying the influenza nucleoprotein peptide sequence. The results also showed an increased efficiency of lysis as a result of adding the tat sequence.

### Example 2

To illustrate the utility of recombinant antibodies to direct CTLs to cancer cells, a recombinant antibody was generated which was specific for the MBr1 antigen (Menard *et al*., 1983 Cancer Research *43*, 1295-1300) on certain cancer cells. The sequence of the immunoglobulin variable regions for the mouse antibody (Orlandi *et al.,* 1989 Proc. Natl. Acad. Sci. USA *86*, 3833-3837) was used as a starting point and was generated using standard methods by site-directed mutagenesis of the variable regions of plasmids M13-VHPCR1 and M13-VKPCR1 (Orlandi *et al*., loc. cit.). The MBr1 M13-VHPCR1 and M13-VKPCR1 plasmids were digested with *Pst*I/*BstE*II and *Pvu*II/*Bcl*I respectively and the small fragments were gel purified. These fragments were then PCR amplified using the Pharmacia Phage Antibody System and individual phage clones were screened by ELISA for binding to MCF7 breast cancer cells (ECACC No. 86012803) using the anti-M13 antibody. Soluble scFvs were prepared from positive phage by infection of HB2151 cells. Subsequently, P53 or FLU pentameric sequences were cloned into MBr1-specific phage with or without the tat peptide as in example 1 above.

Human cytotoxic T lymphocyte (CTLs) specific for the flu peptide GILGFVFTL were obtained from a normal HLA-A2 donor and were maintained as described by Bednarea *et al*., (1991 J. Immunological Methods *139*, 41-47). Testing of antibodies for ability to induce CTL activity against target MCF7 cells was as for example 1 with effector:target ratios of 40:1, 20:1 and 10:1. Results were as follows;

| **Treatment** | **% Specific** ^{**51**}**Cr Release at E/T Ratio** | | |
|---|---|---|---|
| | **40:1** | **20:1** | **10:1** |
| Anti MBr1-FLU | 13 | 6 | 5 |
| Anti MBr1-FLU/tat | 37 | 20 | 13 |
| Anti MBr1-p53/tat | 10 | 7 | 5 |
| Anti MBr1 (no peptides) | 8 | 7 | 6 |
| flu peptide only | 68 | 45 | 26 |

These results demonstrated the induction of cell lysis following incubation with antibodies specific for MBr1 molecules and carrying the flu and tat peptide sequences but not antibodies carrying the p53 peptide sequence. Omission of the tat sequence gave only a marginal increase in cell lysis by the scFv with the flu peptides.

### Example 3:

In another example to illustrate the invention, the antibody of Figure 1 could be generated to include tandem-repeated multiple copies of the CTL-inducing nonapeptides derived from the genes, MAGE-1, 2 and 3 (van den Eynde *et al.,* 1989 Int. J. Cancer *44*, 643) with suitable spacing provided by short runs of non-immunodominant amino acids between the immunodominant peptides and with adjacently provided translocating domains such as from the HIV-1 tat protein. This could then be administered to a patient types as HLA-A1 harbouring a cancer with an internalising antigen (such as Lewis-Y) such that the antibody will internalise into the tumour and will potentially present the MAGE nonapeptides to the immune system. This may then activate MAGE-specific CTLs which will then lyse the tumour: alteratively, the patient could be preimmunised with MAGE peptides or an antibody such as in example 3 in order to expand and preactivate MAGE-specific CTLs.

### Example 4:

As an alternative to example 1, antibodies could be generated in the same manner but containing class-II MHC restricted peptides such as the tetanus toxin peptide P2 (Panina-Bordignon *et al.,* 1989 Eur. J. Immunol. *19*, 2237) and excluding the cytoplasm translocating domain. Such antibodies would be administered to a cancer patient with an appropriate HLA-DR type for presentation of P2. If the antibody is again specific for an internalising antigen on the cancer cells, then the antibody will internalise into the tumour and will potentially present the P2 peptides to the immune system. This may then activate P2-specific T helper cells to release lymphokines and to activate B cells and also may activate CTLs which will then lyse the tumour; in addition, a greater antitumour response may be generated if the patient is preimmunised with P2 peptide or an antibody such as in example 3 in order to expand and preactivate P2-specific CTLs.

### Example 5:

As alternatives to examples 1 and 2, antibodies could be generated in the same manner but with specificity for an internalising antigen on professional antigen-presenting cells, such as specificity for MHC class II antigen or specificity for surface-Ig on B cells, and including peptides presented by, for example cancer cells (e.g. for MAGE 1-3, a high proportion of human malignant melanoma cancers). If administered to a cancer patient with an appropriate MHC type for presentation of the peptides carried within the antibody, these should be processed and presented to the immune system by the antigen-presenting cells which would then stimulate an immune response against the cancer antigen represented by the peptides presented. In addition, antibodies from examples 1 and 2 might be used in combination with the type of antibody of this example in order to efficiently present the antigen on the target cancer cells for effective activation of T cells.

### Example 6:

As alternatives to examples 1 to 3, antibodies could be generated in the same manner for use in autoimmune diseases where the autoimmune immunodominant peptides are known. For example, T cells involved in the deterioration of normal tissue in the disease could be deleted or inhibited by creating an antibody specific for an internalising antigen on a professional antibody producing cell as in example 3 or by targeting the cells of the tissues subject to deterioration (e.g. thyroid cells via the TSH receptor for Graves Thyroiditis). In each case, the antibody would include multiple copies of the autoimmune immunodominant peptide to induce tolerance or would include altered peptides which, upon presentation, can bind to but not activate the deleterious T-cell thus inhibiting autoimmunity.

### Example 7:

As an alternative to example 3, antibodies could be generated with specificity for an internalising antigen on professional antigen-presenting cells, such as specificity for MHC class II antigen or specificity for surface-Ig on B cells, and including immunodominant peptides derived from infectious disease agents (e.g. the HIV V3-loop epitope conserved in a high proportion of HIV isolates). If administered to an infected patient with an appropriate MHC type for presentation of the peptides carried within the antibody, these should be processed and presented to the immune system by the antigen-presenting cells which would then stimulate an immune response against the infectious agent. Indeed, antibodies used in this manner might constitute effective vaccine agents as an alternative to vaccines comprising peptides or live/attenuated infectious agents which might not be efficiently processed by antigen-presenting cells.

### Example 8:

As an alternative to example 3. antibodies could be generated with specificity for an internalising antigen on professional antigen-presenting cells and including immunodominant peptides derived from allergens which normally induce a TH2 allergenic response. If administered preferably to the mucosal surfaces of a sensitised patient with an appropriate MHC type for presentation of the peptides carried within the antibody, these should be processed and presented to the immune system by the antigen-presenting cells which would then stimulate the induction of TH1 cells in preference to TH2 cells thus alleviating the allergenic response to the allergen. Indeed, antibodies used in this manner might constitute effective vaccine agents as an alternative to vaccinating with allergen preparations which might not be efficiently processed by antigen-presenting cells.

### Example 9:

As an alternative to example 3, antibodies could be generated with specificity for an internalising antigen on professional antigen-presenting cells and including immunodominant peptides derived from autoimmune antigens which normally induce a T helper response. If administered to an afflicted patient with an appropriate MHC type for presentation of the peptides carried within the antibody, these should be processed and presented to the immune system by the antigen-presenting cells which would then induce tolerance in the T helper clones responsible for the autoimmune response. Indeed, antibodies used in this manner might constitute effective alternatives to peptide vaccines.

## Claims

1. A chimaeric polypeptide comprising: a binding portion comprising an immunoglobulin molecule or an effective portion thereof having specific binding affinity for a eukaryotic target cell surface component, and an effector portion consisting of one or more copies of an immunogenic peptide; whereby binding of the polypeptide to the cell surface component induces internalisation of at least the effector portion so as to allow the immunogenic peptide to be presented by MHC molecules on the target cell surface.

2. A polypeptide according to claim 1, wherein the cell surface component is an antigen or a receptor molecule.

3. A polypeptide according to any one of the preceding claims, wherein after internalisation the peptide is presented on the surface of the target cell in association with class I MHC antigen so as to modulate a CTL response.

4. A polypeptide according to any one of the preceding claims wherein after internalisation the peptide is presented on the surface of the target cell in association with class II MHC antigen so as to modulate a T helper cell response.

5. A polypeptide according to any one of the preceding claims, wherein the effector portion comprises one or more immunodominant T cell peptide epitopes.

6. A polypeptide according to any one of the preceding claims, wherein the effector portion comprises a number of repeats of the same peptide capable of exerting an immunomodulatory effect.

7. A polypeptide according to any one of the preceding claims, wherein the effector portion comprises a plurality of different peptides, such that the different peptides are capable of being presented by respective MHC antigens of a different haplotype.

8. A polypeptide according to any one of the preceding claims, wherein the cell surface component is selected from the group consisting of: MHC class I antigen; MHC class II antigen; FcRI receptor; B cell surface immunoglobulin; Lewis Y antigen; TSH receptor; and the MBr1 antigen.

9. A polypeptide according to any one of the preceding claims, wherein the effector portion comprises a peptide unit(s) selected from the group consisting of: MAGE 1, 2 or 3 antigens; tetanus toxin P2 peptide; the HIV-V3 loop epitope; the p53 anti-oncogene; protein; influenza virus matrix protein; and influenza virus nucleoprotein.

10. A polypeptide according to any one of the preceding claims, further comprising a signal directing the peptide unit(s) to a particular cellular compartment.

11. A polypeptide according to any one of the preceding claims, comprising a translocation portion derived from the translocation domain of a bacterial exotoxin or HIV tat protein, or the endosome-disrupting function of an adenovirus.

12. A polypeptide according to claim 11, wherein the signal is derived from the translocation domain of *Pseudomonas* exotoxin.

13. A polypeptide according to any one of the preceding claims, wherein the target cell is a "professional" antigen presenting cell (APC).

14. A polypeptide according to any one of claims 1 to 12, wherein the target cell is an aberrant, virus-infected or otherwise diseased cell.

15. A polypeptide according to claim 14, wherein the target cell is a tumour cell.

16. A polypeptide according to claim 14 or 15, wherein the cell surface component is a tumour-associated antigen.

17. A vaccine for stimulating an immune response, comprising an effective amount of a polypeptide in accordance with any one of the preceding claims, together with a physiologically acceptable carrier substance.

18. Use of a polypeptide in accordance with any one of claims 1 to 16 in the manufacture of a medicament for modulating the immune response of a human or animal subject.

19. The use according to claim 18, wherein administering the medicament causes the target cell to present on its surface, together with an MHC antigen, an amino acid sequence which would not normally be presented by the target cell.

20. The use according to claim 19, wherein administering the medicament causes the target cell to present a CTL epitope which is foreign to the subject.

21. A polypeptide according to any of the claims 1 to 16 for use in medicine.

## Patentansprüche

1. Chimäres Polypeptid, umfassend: einen bindenden Abschnitt, enthaltend ein Immunglobulinmolekül oder einen wirksamen Teil davon, mit spezifischer Bindungsaffinität für eine eukaryontische Targetzell-Oberflächenkomponente, und einen Effektor-Abschnitt, bestehend aus einer oder mehreren Kopien eines immunogenen Peptids; wobei durch Bindung des Polypeptids an die Zelloberflächenkomponente die Internalisierung zumindest des Effektor-Abschnitts induziert wird, so daß das immunogene Peptid von MHC-Molekülen auf der Oberfläche der Targetzelle präsentiert werden kann.

2. Polypeptid nach Anspruch 1, bei dem es sich bei der Zelloberflächenkomponente um ein Antigen oder ein Rezeptormolekül handelt.

3. Polypeptid nach einem der vorhergehenden Ansprüche, bei dem das Peptid nach der Internalisierung in Assoziation mit einem Klasse-I-MHC-Antigen auf der Oberfläche der Targetzelle präsentiert wird, so daß eine CTL-Reaktion moduliert werden kann.

4. Polypeptid nach einem der vorhergehenden Ansprüche, bei dem das Peptid nach der Internalisierung in Assoziation mit einem Klasse-II-MHC-Antigen auf der Oberfläche der Targetzelle präsentiert wird, so daß eine T-Helferzellen-Reaktion moduliert werden kann.

5. Polypeptid nach einem der vorhergehenden Ansprüche, bei dem der Effektor-Abschnitt ein oder mehrere immundominante T-Zellpeptid-Epitope enthält.

6. Polypeptid nach einem der vorhergehenden Ansprüche, bei dem der Effektor-Abschnitt mehrere Wiederholungen des gleichen Peptids, das einen immunmodulierenden Effekt ausüben kann, enthält.

7. Polypeptid nach einem der vorhergehenden Ansprüche, bei dem der Effektor-Abschnitt mehrere verschiedene Peptide enthält, derart, daß die verschiedenen Peptide von den entsprechenden MHC-Antigenen eines unterschiedlichen Haplotyps präsentiert werden können.

8. Polypeptid nach einem der vorhergehenden Ansprüche, bei dem die Zelloberflächenkomponente aus der folgenden Gruppe ausgewählt ist: MHC-Klasse-I-Antigen; MHC-Klasse-II-Antigen; FcRI-Rezeptor; B-Zellen-Oberflächenimmunglobulin; Lewis-Y-Antigen; TSH-Rezeptor und MBrl-Antigen.

9. Polypeptid nach einem der vorhergehenden Ansprüche, bei dem der Effektor-Abschnitt eine aus der folgenden Gruppe ausgewählte Peptideinheit(en) enthält: MAGE-1-, -2- oder -3-Antigen; Tetanustoxin-P2-Peptid; HIV-V3-Schleifen-Epitop; p53-Anti-Onkogen-Protein; Matrixprotein des Influenzavirus; und Nukleoprotein des Influenzavirus.

10. Polypeptid nach einem der vorhergehenden Ansprüche, weiterhin enthaltend ein Signal, mit dem die Peptideinheit(en) auf ein bestimmtes Zellkompartiment gerichtet wird.

11. Polypeptid nach einem der vorhergehenden Ansprüche, enthaltend einen Translokationsabschnitt, der sich von der Translokationsdomäne eines bakteriellen Exotoxins oder eines HIV-tat-Proteins oder der Endosomenunterbrechungsfunktion eines Adenovirus ableitet.

12. Polypeptid nach Anspruch 11, bei dem sich das Signal von der Translokationsdomäne des *Pseudomonas*-Exotoxins ableitet.

13. Polypeptid nach einem der vorhergehenden Ansprüche, bei dem es sich bei der Targetzelle um eine "professionelle" Antigen-präsentierende Zelle (APC) handelt.

14. Polypeptid nach einem der Ansprüche 1 bis 12, bei dem es sich bei der Tragetzelle um eine anormale, virusinfizierte oder in anderer Weise erkrankte Zelle handelt.

15. Polypeptid nach Anspruch 14, bei dem es sich bei der Tragetzelle um eine Tumorzelle handelt.

16. Polypeptid nach Anspruch 14 oder 15, bei dem es sich bei der Zelloberflächenkomponente um ein tumorassoziiertes Antigen handelt.

17. Impfstoff zur Stimulierung einer Immunreaktion; enthaltend eine wirksame Menge eines Polypeptids nach einem der vorhergehenden Ansprüche zusammen mit einem physiologisch unbedenklichen Trägerstoff.

18. Verwendung eines Polypeptids nach einem der Ansprüche 1 bis 16 zur Herstellung eines Medikaments zur Modulation der Immunreaktion eines Menschen oder Tieres.

19. Verwendung nach Anspruch 18, wobei durch die Verabreichung des Medikaments die Targetzelle dazu veranlaßt wird, auf ihrer Oberfläche zusammen mit einem MHC-Antigen eine Aminosäuresequenz zu präsentieren, die normalerweise nicht von der Targetzelle präsentiert werden würde.

20. Verwendung nach Anspruch 19, wobei durch die Verabreichung des Medikaments die Targetzelle dazu veranlaßt wird, ein CTL-Epitop zu präsentieren, das dem Patienten fremd ist.

21. Polypeptid nach einem der Ansprüche 1 bis 16 zur Verwendung in der Medizin.

## Revendications

1. Polypeptide chimère comprenant: une partie liante comprenant une molécule d'immunoglobuline ou une partie efficace de celle-ci possédant une affinité de liaison spécifique pour un constituant de la surface d'une cellule cible eucaryote, et une partie effectrice constituée d'une ou plusieurs copies d'un peptide immunogène; par lequel la liaison du polypeptide au constituant de la surface de la cellule provoque l'internalisation d'au moins la partie effectrice de manière à permettre au peptide immunogène d'être présenté par des molécules de CMH sur la surface de la cellule cible.

2. Polypeptide selon la revendication 1, dans lequel le constituant de la surface de la cellule est un antigène ou une molécule réceptrice.

3. Polypeptide selon l'une quelconque des revendications précédentes, dans lequel, après l'internalisation, le peptide est présenté sur la surface de la cellule cible en association avec un antigène CMH de classe I de manière à moduler une réponse des CTL.

4. Polypeptide selon l'une quelconque des revendications précédentes, dans lequel, après l'internalisation, le peptide est présenté sur la surface de la cellule cible en association avec un antigène CMH de classe II de manière à moduler une réponse des lymphocytes T auxiliaires.

5. Polypeptide selon l'une quelconque des revendications précédentes, dans lequel la partie effectrice comprend un ou plusieurs épitopes peptidiques de lymphocyte T immunodominant.

6. Polypeptide selon l'une quelconque des revendications précédentes, dans lequel la partie effectrice comprend un nombre de répétitions du même peptide capable d'exercer un effet immunomodulant.

7. Polypeptide selon l'une quelconque des revendications précédentes, dans lequel la partie effectrice comprend une pluralité de peptides différents, de telle sorte que les peptides différents soient capables d'être présentés par les antigènes CMH respectifs d'un haplotype différent.

8. Polypeptide selon l'une quelconque des revendications précédentes, dans lequel le constituant de la surface de la cellule est choisi dans le groupe constitué par un antigène CMH de classe I; un antigène CMH de classe II; un récepteur FcRI; une immunoglobuline de surface de lymphocyte B; un antigène Y de Lewis, un récepteur TSH; et l'antigène MBrl.

9. Polypeptide selon l'une quelconque des revendications précédentes, dans lequel la partie effectrice comprend un ou des motifs peptidiques choisis dans le groupe constitué par: les antigènes MAGE 1, 2 ou 3; le peptide P2 de la toxine tétanique; l'épitope boucle HIV-V3; la protéine de l'anti-oncogène p53; la protéine matricielle du virus de la grippe; et la nucléoprotéine du virus de la grippe.

10. Polypeptide selon l'une quelconque des revendications précédentes, comprenant en outre un signal dirigeant le ou les motifs peptidiques vers un compartiment cellulaire particulier.

11. Polypeptide selon l'une quelconque des revendications précédentes, comprenant une partie de translocation dérivée du domaine de translocation d'une exotoxine bactérienne ou d'une protéine tat de HIV, ou bien la fonction dissociant l'endosome d'un adénovirus.

12. Polypeptide selon la revendication 11, dans lequel le signal est dérivé du domaine de translocation de l'exotoxine *Pseudomonas*.

13. Polypeptide selon l'une quelconque des revendications précédentes, dans lequel la cellule cible est une cellule présentant un antigène (APC)"professionnelle".

14. Polypeptide selon l'une quelconque des revendications 1 à 12, dans lequel la cellule cible est une cellule aberrante, infectée par un virus, ou malade autrement.

15. Polypeptide selon la revendication 14, dans lequel la cellule cible est une cellule tumorale.

16. Polypeptide selon la revendication 14 ou 15, dans lequel le constituant de la surface cellulaire est un antigène associé à une tumeur.

17. Vaccin pour stimuler une réponse immunitaire, comprenant une quantité efficace d'un polypeptide selon l'une quelconque des revendications précédentes, en même temps qu'une substance véhicule physiologiquement acceptable.

18. Utilisation d'un polypeptide selon l'une quelconque des revendications 1 à 16 dans la fabrication d'un médicament destiné à moduler la réponse immunitaire d'un sujet humain ou animal.

19. Utilisation selon la revendication 18, dans laquelle l'administration du médicament entraîne le fait que la cellule cible présente, sur sa surface, en même temps qu'un antigène CMH, une séquence d'acides aminés qui ne serait normalement pas présentée par la cellule cible.

20. Utilisation selon la revendication 19, dans laquelle l'administration du médicament entraîne le fait que la cellule cible présente un épitope de CTL qui est étranger au sujet.

21. Polypeptide selon l'une quelconque des revendications 1 à 16 à utiliser en médecine.
